# EUROPEAN PATENT APPLICATION

(11) **EP 4 431 111 A1**
(43) Date of publication of application: **18.09.2024**
(21) Application number: 23162396.8
(22) Date of filing: 16.03.2023
(51) Int. Cl.: A61K 39/39, A61K 35/747

(54) **PROBIOTIC AS VACCINE IMMUNOADJUVANT**

(71) Applicant: COREE S.r.l., 20122 Milano (IT)
(72) Inventor: Elli, Marina, Bobbio (PC) (IT); Lim, Chong Yoon, Seoul (KR)
(74) Representative: Manfredi, Irene

(57) **Abstract**

The present invention concerns a probiotic for use as immunostimulant, preferably as adjuvant for boosting immune response to a vaccine.

## Description

### FIELD OF THE INVENTION

The present invention concerns the use of a probiotic as immunostimulant for boosting the immune system, in particular as adjuvant for boosting a vaccine's activity.

The probiotic is of strain *Lactobacillus gasseri* (*L. gasseri*) with deposit number LMG P-30998.

### BACKGROUND

Immunization is a success story of global health and development, saving millions of lives every year. Vaccines currently prevents in fact 3.5-5 million deaths every year from diseases like diphtheria, tetanus, pertussis, influenza and measles. Moreover, vaccines are also critical to the prevention and control of infectious disease outbreaks.

Typically, vaccines contain weakened or inactive parts of a pathogenic organism (antigen) that triggers an immune response within the body (immunogen); recently, in response to the covid-19 pandemic outbreak, new vaccines have been developed, that contain the blueprint for making the body of the recipient subject producing antigens, rather than the antigen itself (mRNA vaccines and viral vectors vaccines).

Vaccines typically contain also one or more adjuvants, used to enhance the immune response (immunoadjuvant). In the field of vaccines, the term "(immuno)adjuvant" refers to the ability of a substance to improve (boost) the performances of immunogens, in terms of magnitude, breadth and durability of the desired immune response to vaccine: the boosting effect includes release of higher amounts of antibodies, provision of a longer-lasting protection (the so-called immunogenicity), minimization of the amount of immunogen needed for vaccination, reduction the frequency of booster immunizations needed, and/or improving the immune response also in subjects such as elderly or immunocompromised vaccinees.

Interest in vaccine adjuvants has been growing rapidly, especially after the recent covid-19 pandemic outbreak, raising the attention to the provision of effective vaccination with novel vaccines.

Adjuvants are in fact particularly needed in conjunction with antigen vaccines (also called subunit vaccines), such as the covid-19 vaccines under development, which encompass only the part of the pathogen to which immune recognition results in protection.

In fact, the high purity of antigen vaccines makes adverse events less likely, but it also makes the vaccines less immunogenic and therefore potentially less effective.

Presently available adjuvants include chemical substances, such as aluminium adjuvants (alum), oil-in-water adjuvants, saponin adjuvants, oligodeoxynucleotides (CpG), bacterial lipopolysaccharides (LPS), Pattern recognition receptors (PRRs) agonists. Unfortunately, few adjuvants have sufficient potency and low enough toxicity for clinical use.

General body reaction to vaccines is based on the induction of Th1-type cytokines to produce the pro-inflammatory responses responsible for killing intracellular parasites and for perpetuating autoimmune responses. Interferon gamma (IFNγ) is the main Th1 cytokine. Recent investigation on innate immunity demonstrates that adjuvant activity is initiated from the stimulation on innate immunity and/or inflammasome, resulting in cytokine induction and antigen uptake by monocytes and macrophages.

However, local and/or systemic adverse events are also observed following the administration of an adjuvated vaccine, due to immunological hyperactivation and release of pro-inflammatory cytokines. Adjuvants can then also trigger inflammatory or autoimmune illnesses in genetically susceptible humans. For instance, Alum adjuvant is known to induce a higher incidence of febrile reaction in children than in adults, but also oil adjuvants have been found to induce a higher incidence of adverse events, especially for local reactions, in influenza pandemic vaccines.

Therefore, it is highly desirable to provide new adjuvants that are potent but also safe. Moreover, it is highly desirable to provide new immunostimulant agents, capable of improving the functionality of the immune system, enhancing the intrinsic antimicrobial functions of the immune system, even long after initial stimulation, to achieve the so-called "trained immunity". Trained immunity is a newly emerging concept that refers to the capacity of the innate immune system to have an immune memory and provide long-lasting protection against foreign antigens. Training, supporting or priming the immune system can help to keep the homeostasis of the immune system, or to quickly return to homeostasis, moderating the size or the duration of infections. Trained immune system activates monocytes and macrophages and induces cytokine production. Trained innate cells are therefore considered very relevant in priming/boosting vaccine strategies (Palgen et al. 2021).

Mucosal vaccination, capable of inducing protective immune responses both in the mucosal and systemic immune compartments, has many advantages and is regarded as a blue ocean in the vaccine industry. Mucosal vaccines can offer lower costs, better accessibility, needle-free delivery, and higher capacity of mass immunizations during pandemics. Also, since many pathogens infect the host via the mucosal route such as inhalation, ingestion, or sexual contact, development of vaccine that can both prevent the mucosal invasion of the pathogen at the infection stage and also neutralize the pathogen-derived toxin or inhibit replication of the pathogen within the body at later infection stages are essential to prevent infectious diseases.

Still, among authorised adjuvants only few (Choleric Toxin, Thermolabile Enterotoxin and Chitosan) are categorised as mucosal adjuvant.

In fact, despite the many advantages mentioned above, with the mucosal vaccination strategies still there exist many disadvantages: large doses are needed, due to instability of antigens in the mucosal surfaces, poor immunogenicity (tolerance induction), physical mucosal barriers, etc. Therefore, it is also highly desirable to provide new adjuvants that can boost immune response to mucosal vaccines, being potent and safe.

Therefore, it is also highly desirable to provide new immunoadjuvants that can safely boost the immunogenic activity of mucosal vaccines.

### SUMMARY OF THE INVENTION

The above objects have been achieved by the present invention, concerning the use of a probiotic for boosting immune system, preferably as immunoadjuvant for boosting a vaccine's activity; the probiotic is of strain *Lactobacillus gasseri* (*L. gasseri*) with deposit number LMG P-30998.

The present invention concerns then the use of said probiotic in a method of boosting the immune system, preferably in conjunction with a vaccine, being capable of acting as immunoadjuvant, boosting immune response of a subject to an immunogen.

Said probiotic is in particular capable of boosting the immune system when used in conjunction with a vaccine to give rise to a much stronger immune response than that seen with vaccine only; said probiotic is therefore capable of boosting immunity provided by the vaccine.

Moreover, said probiotic shows improved safety, avoiding or reducing undesired inflammation.

The present invention also concerns compositions or kits comprising the probiotic and a vaccine. The probiotic can thus be formulated in a composition with the vaccine, or it can be provided as a kit comprising the probiotic and the vaccine, wherein the probiotic can be administered separately from the vaccine, before or after vaccination, within a time suitable for achieving the desired immunoadjuvant activity.

Optionally, the probiotic for use according to the invention is in the form of, or formulated within, a food or feed, as well as supplements thereof comprising the probiotic with suitable food or feed additives.

The present invention also concerns compositions or medical devices comprising the probiotic for use in a method of boosting the immune system, preferably as immunoadjuvant in conjunction with a vaccine.

Preferably the probiotic is inactivated, more preferably by heat inactivation.

### BRIEF DESCRIPTION OF FIGURES

The characteristics and the advantages of the present invention will become apparent from the following detailed description, the working examples provided for illustrative and non-limiting purposes, and the annexed figures wherein:

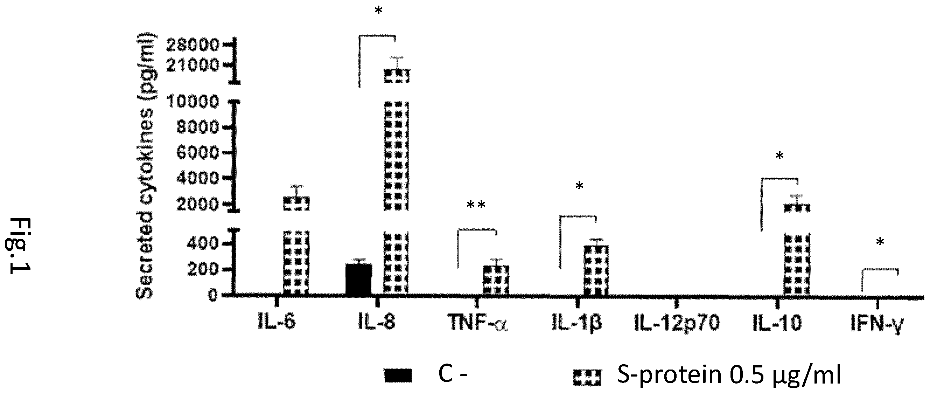

- Figure 1 shows the release of cytokines from human peripheral blood mononuclear cells (hPBMCs), model of systemic vaccination, challenged with S-protein antigen of Sars-CoV-2 (T-test *p<0,05, **p<0,01 vs unchallenged control, C-)

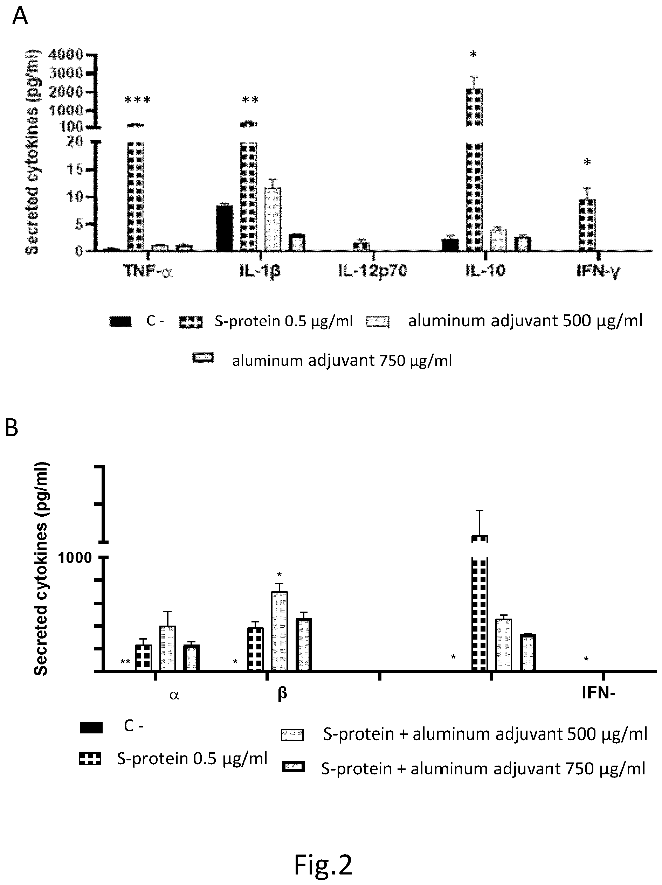

- Figure 2 shows the release of cytokines from hPBMCs challenged with S-protein or aluminum adjuvant (A) (One Way Anova vs unchallenged control, C- *p<0,05, **p<0,01, ***p<0.001), or with both S-protein together with aluminium adjuvant (B) (One Way Anova vs S-protein alone *p<0,05, **p<0,01)

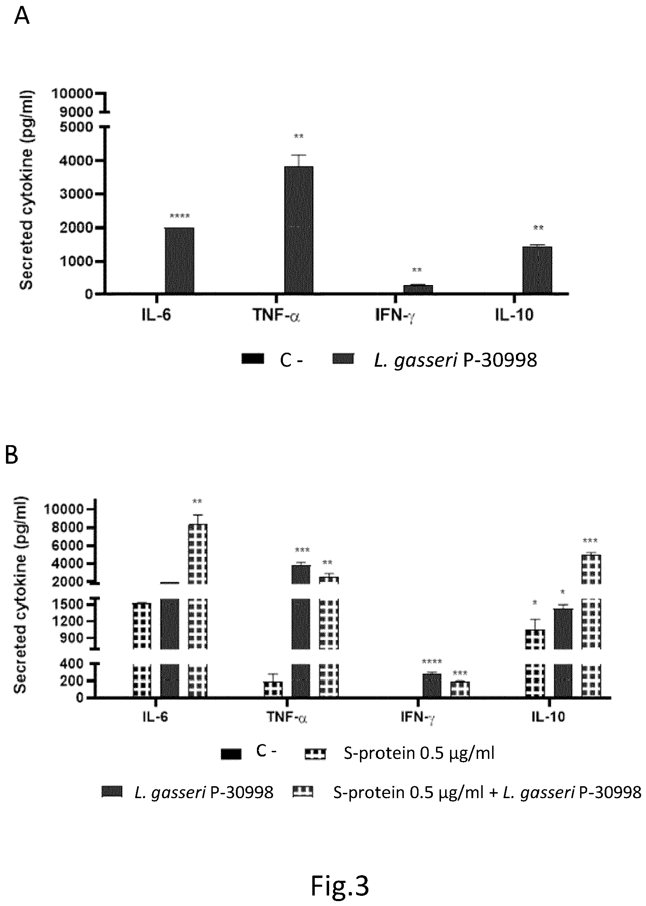

- Figure 3 shows the release of cytokines from hPBMCs exposed to the heat-inactivated probiotic *L.gasseri* LMG P-30998 alone (A) (for each gene, T-test ** p<0.01, *** p<0.001) or as vaccine adjuvant together with S-protein (B) (One Way Anova vs C-*p<0,05, **p<0,01, ***p<0,001, ****p<0,0001)

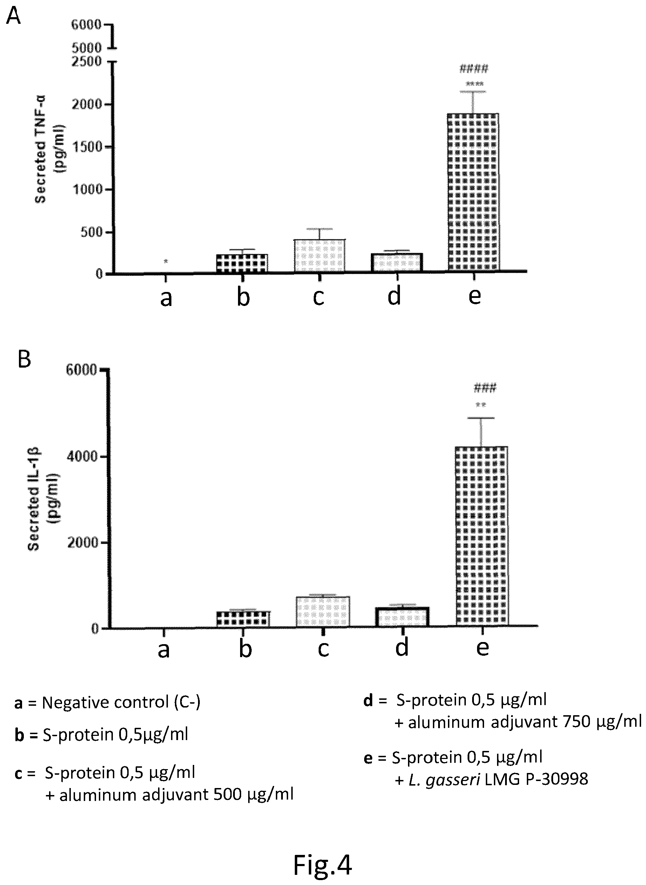

- Figure 4 shows the release of (A) TNF-α and (B) IL-1β cytokines from hPBMCs challenged with S-protein alone or together with aluminium adjuvant or *L.gasseri* LMG P-30998 adjuvants (One Way Anova vs aluminium adjuvant 500µg/ml (^{∗}) or 750µg/ml (#) ^{∗/#}p<0,05, ^{∗∗/##}p<0,01, ^{∗∗∗/###}p<0,001, ^{∗∗∗∗/####}p<0,0001)

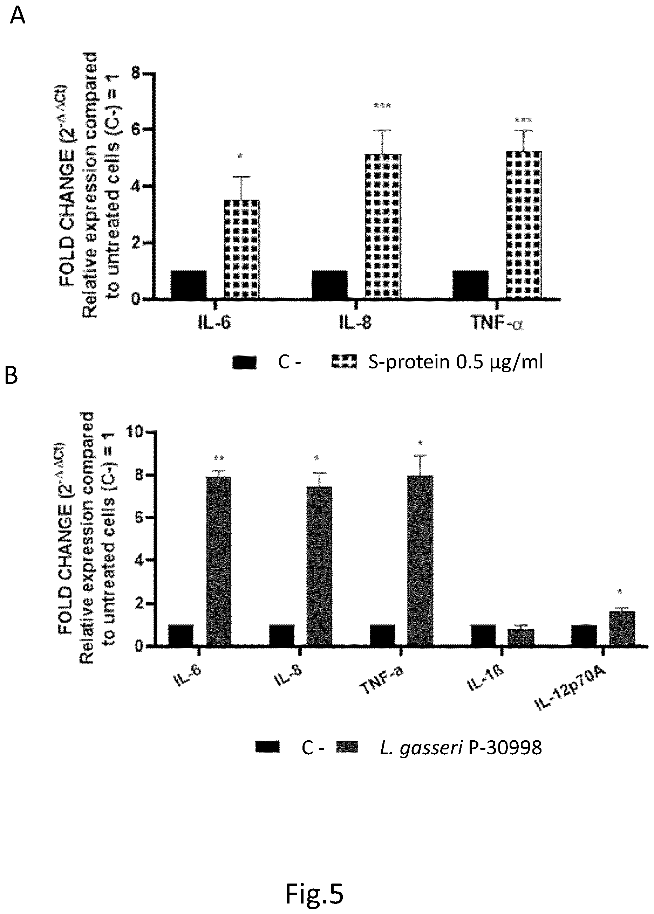

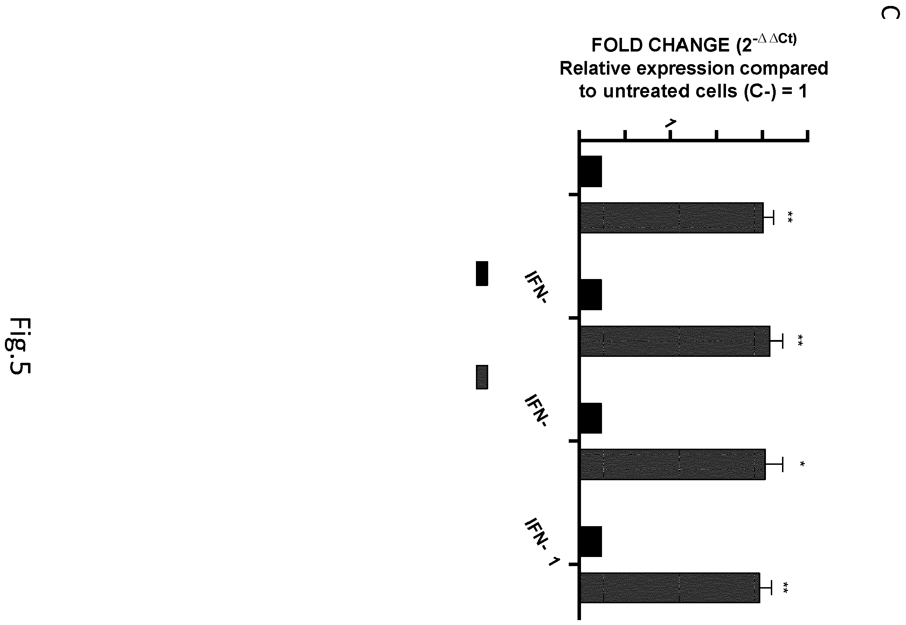

- Figure 5 shows the immune response, in terms of cytokines' relative gene expression by qPCR vs control (C-, unchallenged cells), of Caco-2 intestinal cells, model of mucosal vaccination, challenged with S-protein (A) or with inactivated *L.gasseri* LMG P-30998 (B, C) (For each gene, T-test *p<0,05, *** p<0,001)

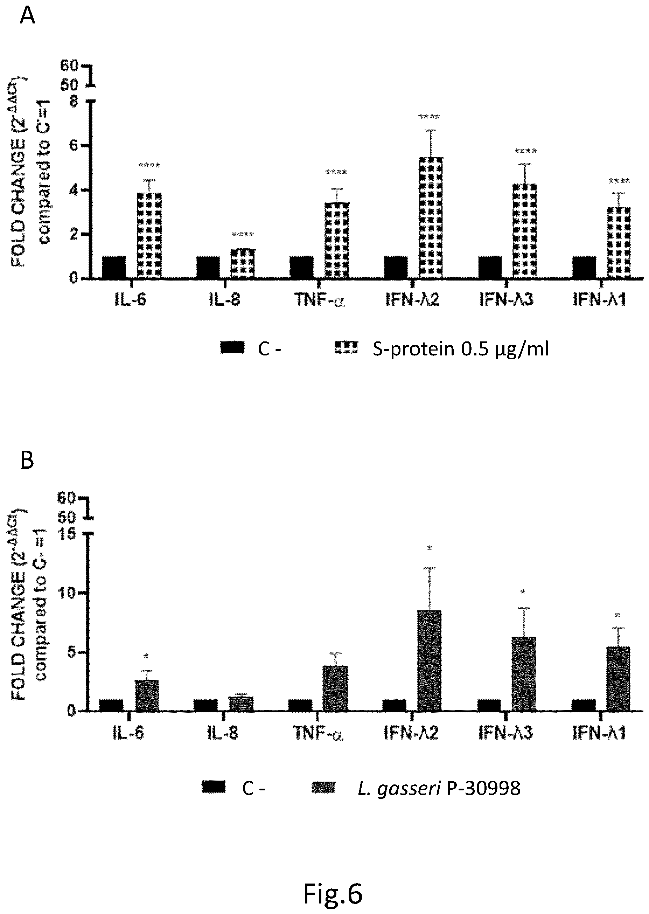

- Figure 6 shows the immune response, in terms of cytokines' relative gene expression vs control (C-, unchallenged cells) by qPCR, of A549 lung cells challenged with (A) S-protein, or with (B) the heat-inactivated probiotic LMG P-30998. (For each gene, T-test * p<0.05, **** p<0.0001)

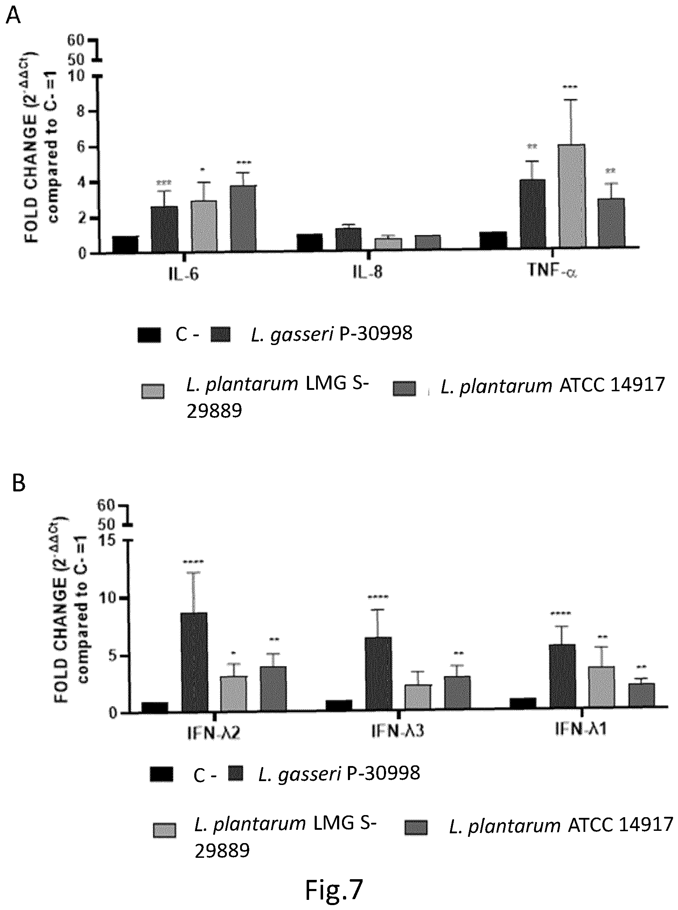

- Figure 7 shows the immune response, in terms of cytokines' relative gene expression vs control (C-, unchallenged cells) by qPCR, of lung cells exposed to different heat-inactivated probiotics: panel A shows the pro-inflammatory cytokines' gene expression, panel B shows the anti-viral cytokines' gene expression. For each gene: One Way Anova vs C- cells *p<0,05, **p<0,01, *** p<0,001, **** p<0,0001

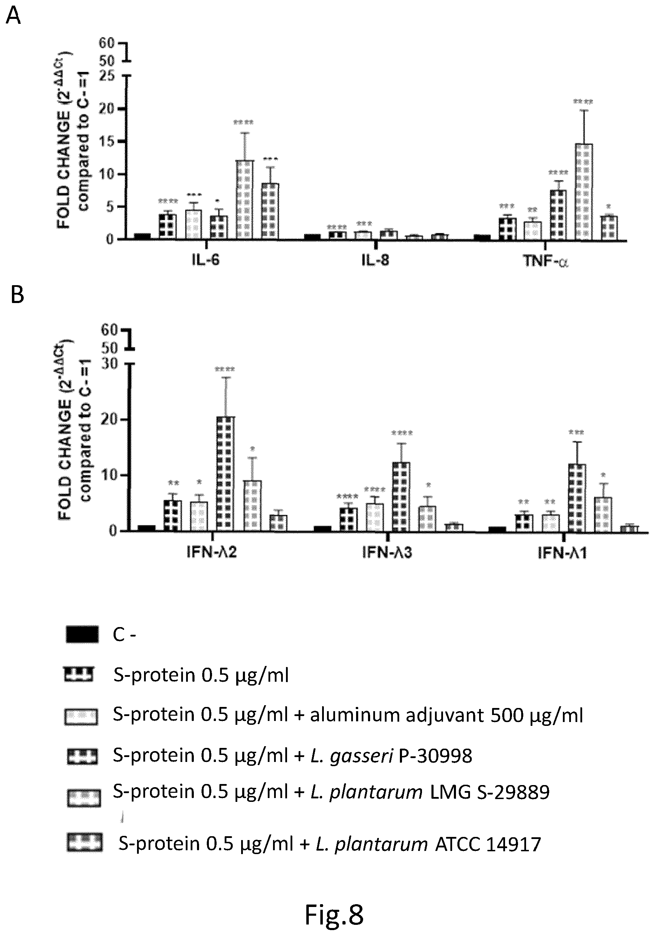

- Figure 8 shows the immune response, in terms of cytokines' relative gene expression vs control (C-, unchallenged cells) by qPCR, of A549 lung cells challenged with S-protein alone, or together with aluminium adjuvant or inactivated probiotic adjuvants (*L. gasseri* LMG P30998 or *L. plantarum*): panel A shows the pro-inflammatory cytokines' gene expression, panel B shows the anti-viral cytokines' gene expression. For each gene: One Way Anova vs C- cells *p<0,05, **p<0,01, *** p<0,001, **** p<0,0001

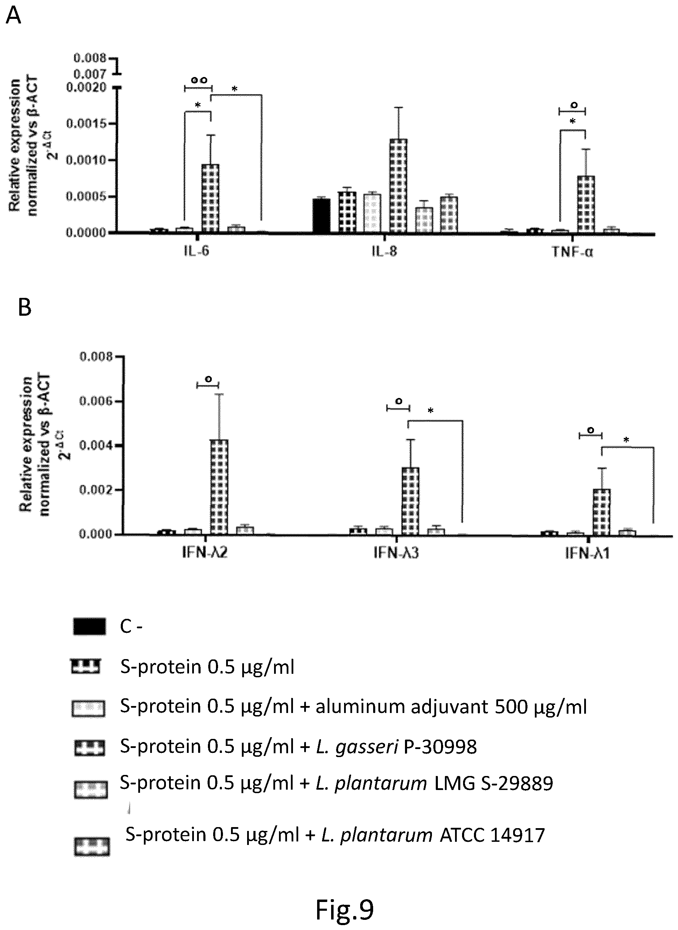

- Figure 9 shows the immune response, by cytokines' gene expression normalized on β-actin, of A549 lung cells challenged with S-protein alone, or together with chemical or probiotic adjuvants: panel A shows the pro-inflammatory cytokines' gene expression, panel B shows the anti-viral cytokines' gene expression. For each gene: One Way Anova vs S-protein + LMG P30998 (*p<0,05). For each gene, T-test S-protein + aluminium adjuvant vs S-protein + LMG P-30998 (° p<0.05 and °° p<0.01)

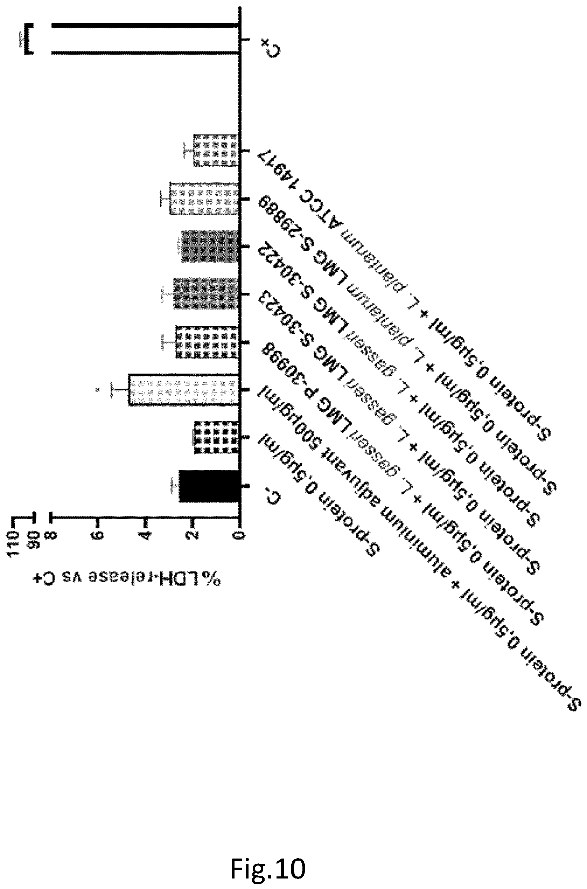

- Figure 10 shows the cytotoxic effect of S-protein alone, or together with chemical or probiotic adjuvants, on A549 cells. Cytotoxicity was evaluated by LDH-release assay. Positive control cells (C+) are set at 100% of cells cytotoxicity. One-way ANOVA vs C-^{∗} p ≤ 0.05.

### DETAILED DESCRIPTION OF THE INVENTION

The subject of the invention is therefore a probiotic bacterial strain (hereafter also indicated simply as "probiotic") for use as immunostimulant.

Preferably, the probiotic is used in a method of boosting immunity of a subject, more preferably as adjuvant in a method of immunization of a subject with a vaccine, said method comprising administering the vaccine to the subject.

The probiotic of the invention is therefore preferably used as adjuvant to boost the immune response of a vaccine.

Probiotics are bacteria that, when administered in adequate amount, are able to exert beneficial effect on the host (FAO, 2002). Microbial strains with proven beneficial properties are available for human consumption as food (e.g., fermented milk) as well as food supplements and even drugs.

Probiotic bacteria are generally found among the genera *Lactobacillus, Streptococcus, Lactococcus* and *Bifidobacterium.* A number of strains from these genera are commonly used for making cheese, yoghurt, and other dairy products.

Probiotics have shown a beneficial effect on different conditions such as lactose malabsorption, acute diarrhoea, antibiotic-associated diarrhoea, allergies, and inflammatory bowel disease.

Limited research suggests that certain probiotic strains may have adjuvant-like functions, enhancing antibody levels produced by certain vaccines. However, so far, no probiotics adjuvants have been proved to be also safe, avoiding or reducing undesired hyperactivation of immune system and release of pro-inflammatory cytokines. Also, health benefit of probiotics is strain-dependent, therefore functional effects demonstrated for one probiotic strain cannot necessarily be extrapolated to other strains.

Inventors have surprisingly identified a probiotic bacterial strain which is capable of acting as immunostimulant, boosting immunity, in particular in response to vaccines, avoiding or reducing undesired hyperactivation of immune system, as ascertainable by the reduced or absent release of pro-inflammatory cytokines, compared to currently available adjuvants.

Advantageously, as it will be also demonstrated in the Examples below, the use of the probiotic strain according to the invention in conjunction with a vaccine surprisingly and unexpectedly provides a synergistic immunogenic effect.

The probiotic used in accordance with the present invention is a probiotic bacterial strain *Lactobacillus gasseri* (*L. gasseri*) previously deposited on 20^{th} November 2018 under Budapest Treaty at BCCM/LMG Bacteria collection (Universiteit Gent Laboratorium voor Microbiologie K.L. Ledeganckstraat, 35- 9000- Gent) with number LMG P-30998.

This strain has in fact shown to exceed other probiotics adjuvants in boosting vaccine's immunogenic activity, with improved safety.

For the purposes of the present invention, with the terms "immunostimulant" activity and "boosting" immunity (or immune system or immune response) it is meant the improvement of functionality of the immune system through the enhancement of trained immunity. The use of the probiotic bacterial strain as immunostimulant is thus meant to indicate its medical use as defined by its immunostimulant function.

Improved functionality of the immune system includes enhanced production of cytokines in various organs, tissues, body fluids (e.g., lung, bronchoalveolus, plasma etc.). For instance, the lymphocyte subpopulations (CD4+ vs CD8+) or the number and functionality of activated NK cells, by specific membrane markers or activation markers, can be assessed by flow cytometry, compared to physiological levels. Also, through methods known in the art, the activation of interferons and the release of cytokines can be evaluated (Netea et al. 2020), in particular, the release of IL-1, IL-12, IL-5- and IL-13, which increase in case of extracellular expulsion of parasites, or of IL-22 and IL-17, involved in immunity against bacteria and fungi, or of IL-12 and IL-18, normally released in the case of intracellular pathogen infections.

For the purposes of the present invention, the terms "adjuvant" or "immunoadjuvant" indicate a substance that enhances (boosts) activity of a vaccine, in particular the immune response to an antigen, that is triggered by the immunogen of the vaccine.

An "adjuvant" or "immunoadjuvant" according to the present invention is a substance that has immunostimulant function in conjunction with a vaccine, enhancing the activity of the latter, in particular enhancing the immune response to an antigen, that is triggered by the immunogen of the vaccine.

The term "vaccine" is known to indicate a biological preparation comprising antigenic material (immunogen), that is capable of stimulating the immune response to a pathogen in a subject. The term "vaccine" is herein used also to indicate a biological preparation that comprises the instructions for producing an immunogen, such as a nucleic acid encoding for an immunogen, capable of making a cell producing the immunogen upon receipt of the vaccine.

Preferably the vaccine, whose activity is boosted by the probiotic adjuvant according to the present invention, is a subunit vaccine.

The term "subunit vaccine" (also "antigen vaccine") refers to a vaccine comprising an antigen being isolated protein, fragment, or carbohydrate, derived from virus, bacteria or any other organism, or being isolated or extracted from an organism, preferably from a pathogenic organism, or being a biological molecule capable of inducing production of the antigen by the vaccine's recipient cells of a subject.

One example of subunit vaccine is the vaccine against hepatitis B, which is composed of the surface proteins of the hepatitis B virus; another example is edible algae vaccines, such as the virus-like particle (VLP) vaccine, against human papillomavirus (HPV), which is composed of the viral major capsid protein. Another example is the hemagglutinin and neuraminidase subunits of the influenza virus.

For the purposes of the present invention, preferably the vaccine is a vaccine against Sars-CoV-2 virus, which is the pathogen responsible of covid-19 disease; more preferably, it is a vaccine comprising the recombinant spike glycoprotein (S-protein) of Sars-CoV-2 or an immunogenic fragment thereof. Alternatively, the vaccine can be a vaccine against Sars-CoV-2 virus that comprises a nucleic acid, RNA or DNA, encoding for the antigenic S-protein or an immunogenic fragment thereof, optionally delivered by a viral vector in the cells of the vaccine's recipient subject.

Therefore, according to the present invention, the subunit vaccine can also be a so-called "RNA vaccine", inducing production of the antigenic protein, or fragments thereof, in the cells of the vaccine's recipient subject, or a "viral vector vaccine" delivering a nucleic acid for producing the antigenic protein, or fragments thereof, in the cells of a subject after administration of vaccine.

Preferably, whole virus or split vaccines are not within the scope of the invention. Preferably, for the purpose of the present invention, the vaccine is a mucosal vaccine. Mucosal vaccines are those administered through mucosal tissues, such as those administered by nasal, oral, ocular, rectal, or vaginal administration.

Mucosal vaccines are very suitable for reducing allergic effects of systemic vaccines, especially in the pediatric field.

Due to this synergistic effect of the probiotic used in the present invention in conjunction with vaccines, mucosal vaccination is made more effective and safer.

It is noted that, according to the present invention, the term "a vaccine" is used also to indicate multivalent vaccines, i.e., vaccines comprising different immunogens for immunization against the same or different pathogens.

Surprisingly, the probiotic used in accordance with the present invention is capable of boosting vaccines' activity also when inactivated.

In preferred aspects of the invention, the probiotic is then inactivated, such as by heat-treatment (tyndallized probiotic). Techniques of inactivation of probiotics are known in the art.

Preferably, the probiotic is inactivated by cycles of thermal treatment at temperature between 65°C and 90°C; for example, the probiotic can be inactivated by a treatment comprising; a) by one-cycle of thermal treatment at 85°C for 30 min; or b) by 3-times repeated cycle of thermal treatment at 65°C for 30 min each.

Inactivated probiotics have in general a higher safety profile compared to alive microorganisms. Therefore, the possibility to achieve the boosting effect also when the probiotics of the invention are inactivated is particularly advantageous.

According to the present invention, the probiotic bacterial strain can be administered before, after, or simultaneously with, the vaccine.

The probiotic can thus be formulated in a composition with the vaccine, or it can be provided in a kit comprising the vaccine for separate administration, before or after vaccination, within a time suitable for achieving the desired immunoadjuvant activity. The present invention thus concerns also vaccine compositions comprising a vaccine with the *L. gasseri* probiotic bacterial strain as adjuvant, preferably *L. gasseri* LMG P-30998, or kits separately comprising said probiotic bacterial strain and the vaccine, wherein the bacterial strain is to be administered before or after vaccination, within a time suitable for achieving the desired immunoadjuvant activity, for example within seconds, minutes or hours before or after vaccination. For instance, the probiotical strain can be administered few minutes to 24 hours, before or after the vaccine, preferably 1-12 hours before or after the vaccine.

In preferred embodiments of the present invention, the composition or kit further includes one or more of further adjuvants, preservatives, stabilizers, inactivators, antibiotics, diluents or other suitable pharmaceutical substances or excipients.

In preferred embodiments, the probiotic bacterial strain is the only adjuvant used in conjunction with the vaccine.

The present invention also concerns a method of immunization of a subject with a vaccine, said method comprising administering the vaccine to the subject and administering a dose of the probiotic adjuvant disclosed herein, that is effective in boosting the immunogenic activity of the vaccine in a subject; wherein the probiotic adjuvant can be administered together with, or before, or after, the vaccine.

The subject can be a human or animal, preferably a mammal.

The term "in conjunction with" referred to the vaccine, is meant to indicate the use of the probiotic as vaccine adjuvant, together with, before, or after, the vaccine. Administration of the probiotic may then be simultaneously with the vaccine, optionally as a composition comprising both probiotic and vaccine, or after or prior to administration of the vaccine. With respect to the use of kits according to the present invention, comprising the vaccine and the probiotic separately, the probiotic is preferably administered on a regular basis, such as once or twice daily, optionally at least one, two, or three weeks prior to vaccination. The probiotic may also be administered after vaccination, such as during a period of one, two, three, or four weeks after the vaccination. In a preferred embodiment, administration of the probiotic is made two weeks prior to vaccination and continued until four weeks after vaccination.

The composition or kit of the invention is thus for use in the treatment or prevention of an infection, preferably a viral infection.

Preferably, the effective dose of the probiotic adjuvant administered to a subject in conjunction with the vaccine is 10⁶⁻10¹² CFU (Colony forming unit).

Preferably, the probiotic bacterial strain is then administered in conjunction with vaccine, in a single dose of 10^{6 -}10¹² CFU, more preferably of 10⁷-10¹⁰ CFU, most preferably 10⁸-10¹⁰ CFU.

Optionally the indicated doses are repeatedly administered for a suitable period of time, such as once or twice daily, optionally at least one, two, or three weeks prior to or after vaccination.

When formulated in a composition with the vaccine, preferably the probiotic adjuvant is present in an amount of 10⁶⁻10¹² CFU, more preferably of 10⁷-10¹⁰ CFU, most preferably 10⁸-10¹⁰ CFU.

The same doses are also suitable for probiotics that are inactivated, such as tyndallized probiotics, according to preferred embodiments of the invention (in that case, the above doses shall be correctly indicated as TFU, total fluorescent units, measured by flow cytometry).

Optionally, the probiotic is in the form of a food or feed, as well as food or feed supplements, with suitable food or feed additives. Food and food supplements are intended for human use. Feed and feed supplements are intended for veterinary use. The use of *L.gasseri* LMG P-30998 probiotic as immunoadjuvant is very advantageous for several reasons: *L.gasseri* appears in the list of QPS-recommended biological agents; no adverse events have been reported with *L. gasseri.* Moreover, it has been surprisingly found that the probiotic of the invention is effective also when inactivated, thus improving further its safety. Its production is not expensive in terms of production; also, due to its high effectiveness it can be used at low dosages: for example, the probiotic can be prepared at a titre of 10¹² TFU/g, so that 0,1-1-10 mg of it can be used to provide a dose of 10⁸⁻10¹⁰ TFU.

The probiotic has in fact shown to provide synergy with vaccines; moreover, said synergy is advantageously specific in relation with the cytokines responsible of the antiviral response (e.g., IFNs), with reduced effect on more pro-inflammatory cytokines. Furthermore, the probiotic elicits the desired immune response in several body districts, such as lungs and intestine, being advantageously available as adjuvant of mucosal vaccines.

It should be understood that all the combinations of preferred aspects of the composition of the invention, formulations and uses of the same, as above reported, are to be deemed as hereby disclosed.

Below are working examples of the present invention provided for illustrative purposes.

### EXAMPLES

### Example 1 - systemic vaccination model set-up

Cytokine induction assays are used to evaluate the immunogenic effect of a vaccine: human blood mononuclear cells (hPBMCs) challenged with S-protein of SARS-CoV-2 have been used to mimic systemic vaccination; the cytokines release has been assessed by ELISA assay.

hPBMCs cells were thawed and plated in a 24 multiwell-plate at the concentration of 1^{∗}10⁶ cell/ml, in RPMI medium added with 10% heat inactivated FBS, 2mM L-glutamine and 50µg/ml gentamicin.

Cells were left undisturbed for 24h and, after this period, the experiment was started.

Anti-SARS-Cov-2 S-protein immune response was tested by stimulating hPBMCs with 0,5µg/ml of S-protein for 24h, then evaluating the secretion of different pro-inflammatory (IL-6, IL-8, TNF-α, IL-1β and IL-12p70) or anti-inflammatory (IL-10) and anti-viral (IFN-γ) cytokines. Each molecule was quantified by ELISA technique, using commercial kit (ThermoFisher Scientific) following manufacture's protocol. Control included cells treated with culture medium alone (C-).

As shown in Fig. 1, S-protein antigen of Sars-CoV-2 (0,5 µg/ml) administration to hPBMCs produced a statistically significant release of IL-8, IL-10, IL-1β and TNF-α. S-protein antigen is thus immunogenic and cytokine release by hPBMCs challenged with the antigen can be used to evaluate the effects of systemic vaccination.

### Example 2 - impact of conventional adjuvants in systemic vaccination

The induction assay of example 1 has been repeated, challenging hPBMCs with S-protein in the presence of a conventional chemical aluminum adjuvant (Imject^{™} Alum adjuvant, from Fisher Scientific, 500 and 750 µg/ml). As shown in Fig.2A administration of aluminum adjuvant alone produced a slight, non-statistically significant, increase in the release of some cytokines (One-way Anova vs C- * p<0,05, ** p<0,01, *** p<0,001).

Control included cells treated with culture medium alone (C-)

As shown in Fig. 2B, the administration of chemical adjuvant together with the vaccine produced a statistically significant increase of IL-1β, of TNF-α cytokines released by hPBMCs, while IL-10 release was reduced. Therefore, IL-1β and TNF-α are taken as reference-cytokines to evaluated adjuvants' activity (One-way Anova vs S-protein * p<0,05, ** p<0,01).

### Example 3 - impact of probiotic adjuvants in systemic vaccination

The induction assay of example 1 has been repeated, challenging hPBMCs with S-protein in the presence of a probiotic adjuvant according to the invention.

The probiotic strains tested in the following examples were inoculated in MRS broth and cultured for 24h at 37°C under microaerophilic atmosphere. On the day of the experiment, bacterial cultures were centrifuged and the pellet washed twice with sterile double distilled water. Bacterial cells were diluted to obtain about 10⁸ CFU/ml in distilled water and they were heat-inactivated at 80°C for 30 minutes. These heat-inactivated cultures (HT) were then diluted in cell line specific medium to obtain a final concentration of 10⁷ CFU/ml and they were co-cultured with cells for 24 hours.

hPBMCs were stimulated with LMG P-30998 probiotic alone in order to evaluate the immune-modulation properties of this heat inactivated strain. As shown in Fig. 3A the heat-inactivated probiotic *L. gasseri* LMG P-30998 induced the statistically significant release of IL-8, IL-10, IL-12p70 and TNF-α. Moreover, the probiotic synergistically triggers cytokine release from cells challenged with the S-protein in the systemic vaccination model (see Fig. 3B).

### Example 4 - comparison of the impact of chemical and probiotic adjuvants in systemic vaccination

The induction assay of Example 1 has been repeated, challenging hPBMCs with S-protein alone or together with the adjuvants, either chemical or probiotic, prepared as in the previous examples. As shown in Fig. 4, the increase in the release of IL-1β and TNF-α (target cytokines) by hPBMCs challenged with the vaccine was significantly higher in the presence of *L. gasseri* LMG P-30998 microbial adjuvant than in the presence of chemical adjuvant (One Way Anova, probiotic vs Imject 500µg/ml (*); probiotic vs Imject 750µg/ml (^{#}).

### Example 5- mucosal (intestine) vaccination model set-up

A cytokine induction assay (ELISA) is used to evaluate the immunogenic effect of a vaccine: human intestinal adenocarcinoma cells (Caco-2) challenged with 0,5 µg/ml of S-protein antigen of SARS-CoV-2 have been used to mimic mucosal vaccination. Caco-2 cells represent the conventional model of intestinal cell line usually used *in vitro* to study the impact of drugs as well as of probiotics, orally ingested, on the intestinal mucosa in humans.

Human intestinal epithelial cells Caco-2 were grown in DMEM medium + 10% heat inactivated FBS and gentamicin 50µg/ml and maintained at 37°C in 5% CO2.

For the experiment set-up, Caco-2 cells were seeded 3×10⁵cells/ml in 24-well plate for 48h. Once cells reached confluence, they were washed with HBSS twice and leaved undisturbed for 1hour in DMEM medium without antibiotic and FBS. Then, Caco-2 cells were stimulated with 0.5µg/ml SARS-Cov-2 S-protein for 4h. At the end of this incubation period, RNA extraction followed by real-time PCR was performed in order to evaluate IL-6, IL-8, TNF-α, IL-1β, IL-12p70, IL-10, IFN-λ3, IFN-λ2 and IFN-λ1 gene expression. All the reagents used for gene expression analysis (RNA extraction kit, retro-transcriptase kit, SybrGreen mix and primers), in this and following assays, were bought by Biorad and all the procedures were performed following manufacturer's instructions. As shown in Fig. 5A, S-protein antigen of Sars-CoV-2 administered to Caco-2 cells produced a statistically significant release of IL-6, IL-8 and TNF-α cytokines.

### Example 6 - impact of probiotic in intestinal mucosal vaccination model

The induction assay of example 5 has been repeated challenging cells with S-protein or in the presence of 10⁷ CFU of the heat-inactivated form of *L. gasseri* LMG P-30998, prepared as in example 3.

As shown in Figs. 5B and C, the immune response of Caco-2 exposed to the heat-inactivated probiotic *L. gasseri* LMG P-30998 induced a statistically significant release of several cytokines.

### Example 7 -mucosal (oral spray) vaccination model set-up

Human lung epithelial cells A549 were grown in F-12K (Kaighn's Modification of Ham's F-12) medium + 10% heat inactivated FBS and gentamicin 50µg/ml and maintained at 37°C in 5% CO₂.

Epithelial cell line A549 is from human lung and it is typically used to study *in vitro* the impact of a substance orally sprayed. Mucosal vaccines can be, in fact, administered in form of pills, capsules of sticks to be ingested as well as suppositories to be introduced locally or even as nasal/oral spray to be applied directly on oral mucosa or diffused in the environment.

For the experiment set-up, A549 cells were seeded 3×10⁵cells/ml in 24-well plate for 24h. Once cells reached confluence, they were washed with HBSS twice and leaved undisturbed for 1hour in F-12K medium without antibiotic and FBS.

Then, A549 cells were challenged with S-protein of SARS-CoV-2, to mimic mucosal vaccination.

The expression level of the pro-inflammatory cytokines IL-6, IL-8, TNF-α and of the anti-viral IFN-λ2, IFN-λ3 and IFN-λ1 were performed. Controls included cells treated with culture medium alone (C-) or cells stimulated with 0.5µg/ml S-protein alone.

As shown in Fig. 6A, the immune response of lung cells treated with COVID S-protein increased the expression of the genes coding for IL-6, IL-8, TNF-alfa and IFNs cytokines, very relevant to boost antiviral immune reaction. For each gene: T-test, **** p<0,0001 (n=26).

### Example 8 - impact of probiotic in model of mucosal vaccination by oral spray

A549 cells, prepared as in Example 7, were stimulated with heat inactivated probiotic strain (used at a final concentration of about 10⁷CFU/ml in order to maintain MOI 1: 100 cells:bacteria) for 24h. At the end of this incubation, RNA extraction and gene expression analysis of the pro-inflammatory cytokines IL-6, IL-8, TNF-α and of the anti-viral IFN-λ2, IFN-λ3 and IFN-λ1 was performed by real-time PCR.

Controls included cells treated with culture medium alone (C-).

As sown in Fig. 6B, the immune response of lung cells treated with inactivated *L.gasseri* LMG P-30998 results in increased expression of IL-6 and IFNs by lung cells challenged with the bio-based adjuvant. For each gene, T-test *p<0,05.

### Example 9 - impact of different probiotics in model of mucosal vaccination by oral spray

The assay of Example 8 was repeated, with different heat inactivated probiotic strains (10⁷ CFU/ml in order to maintain MOI 1:100 cells:bacteria) for 24h. At the end of this incubation, RNA was extracted and gene expression analysis was performed by real-time PCR. Controls included cells treated with culture medium alone (C-).

As shown in Fig. 7A and 7B, lung cells treated with inactivated *L. gasseri* LMG P-30998 strain unexpectedly show a much higher expression of anti-viral cytokines (see Fig. 7B, expression of IFNs) and a lower expression of pro-inflammatory cytokines (see Fig. 7A, expression of IL-6 and TNF-α), compared to those treated with different Lactobacillus strains. For each gene, One Way Anova vs C- *p<0,05, **p<0,01, *** p<0,001

### Example 10 - impact of chemical and probiotic adjuvants in model of mucosal vaccination by oral spray

The assay of example 8 was repeated, in lung cells challenged with 0.5µg/ml of S-protein antigen, in the presence of 500µg/ml of classical Imject^{™} Alum Adjuvant (Thermofisher Scientific) or in the presence of different probiotic adjuvants.

Controls include cells treated with culture medium alone (C-).

The immune boosting effect obtained with inactivated *L. gasseri* was comparable with that of the chemical adjuvant but with lower pro-inflammatory profile (see Fig. 8A) and with a stronger induction of the antiviral response (see Fig. 8B). Also, the immune boosting effect of *L. gasseri* adjuvant exceed that of *L. plantarum* probiotics in terms of the desired modulation of anti-viral and pro-inflammatory cytokines. For each gene, One Way Anova vs C- cells *p<0,05, **p<0,01, *** p<0,001, **** p<0,0001

Fig. 9 shows the immune response normalized on the expression of the house keeping gene beta-actin (β-ACT).

### Example 11- cytotoxicity assay

The cytotoxicity of all the conditions tested was evaluated on A549 cells by the LDH activity assay. Cells were seeded in 96 well plate at a density of 3×10⁴cells/well: they were leaved at 37°C for 24h and then they were co-cultured with S-protein alone or in the presence of aluminium-based adjuvant or heat-inactivated probiotic strains for 24h. At the end of this incubation period, the release of LDH in the supernatants was evaluated by commercial colorimetric kit following manufacturer's instruction. The control included medium alone used as blank. The spectrophotometric absorbance was measured at 450nm wavelength using a microplate reader.

The maximal release of LDH was obtained after treating cells with 1% Triton X-100 (C+, positive control) for 10 minutes at room temperature. Also, the chemical adjuvant resulted significantly different compared to untreated cells, showing cytotoxicity (see Fig. 10).

## Claims

1. A probiotic bacterial strain *Lactobacillus gasseri* (*L. gasseri*) with deposit number LMG P-30998 for use as immunostimulant.

2. The probiotic bacterial strain for use of claim 1 as vaccine adjuvant in a method of immunization of a subject with a vaccine.

3. The probiotic bacterial strain for use of claim 2 wherein the method of immunization comprises administering the vaccine to the subject and administering the probiotic bacterial strain before, after, or simultaneously with, the vaccine.

4. Vaccine composition or kit comprising a vaccine and a probiotic bacterial strain *L*. *gasseri* with deposit number LMG P-30998.

5. The probiotic bacterial strain for use according to claims 1-3 or the vaccine composition or kit of claim 4, wherein the vaccine is a subunit vaccine and/or wherein the vaccine is a mucosal vaccine.

6. The probiotic bacterial strain for use according to claims 1-3, 5, or the vaccine composition or kit or claims 4-5, wherein the vaccine is a Sars-CoV-2 vaccine.

7. The probiotic bacterial strain for use according to claims 1-3, 5-6, or the vaccine composition or kit or claims 4-6, wherein the probiotic is formulated in a food or in a feed, or supplements thereof.

8. The probiotic bacterial strain for use according to claims 1-3,5-7, or the vaccine composition or kit or claims 4-7, wherein the probiotic is inactivated, preferably by heat.
